# EUROPEAN PATENT SPECIFICATION

(11) **EP 0 942 648 B1**
(45) Date of publication and mention of the grant of the patent: **16.01.2002**
(21) Application number: 97948897.0
(22) Date of filing: 06.11.1997
(51) Int. Cl.: A01M 1/20, A61L 9/03

(54) **ADJUSTABLE-INTENSITY HEATING DEVICE FOR THE EVAPORATION OF CHEMICAL SUBSTANCES**
EINSTELLBARE HEIZEINRICHTUNG ZUM VERDAMPFEN VON CHEMISCHEN SUBSTANZEN
DISPOSITIF DE CHAUFFAGE A INTENSITE REGLABLE, QUI EST DESTINE A FAIRE EVAPORER DES SUBSTANCES CHIMIQUES

(30) Priority: 07.11.1996 IT MI960733 U
(43) Date of publication of application: 22.09.1999
(73) Proprietor: ZOBELE INDUSTRIE CHIMICHE S.p.A., I-38100 Trento (IT)
(72) Inventor: ZOBELE, Franco, I-38100 Trento (IT)
(74) Representative: Faggioni, Giovanmaria, Dr.
(86) International application number: EP9706175
(87) International publication number: WO9819526

(56) References cited:
- EP-A- 0 511 853
- EP-A- 0 736 248
- US-A- 4 769 528

## Description

Heating devices for the evaporation of chemical substances, such as insecticides or perfumed essences, are well known. Two basic types of such heating devices are manufactured: a first type in which heating of a support plate impregnated with the desired active ingredient is performed; a second type in which a wick is provided, said wick being partially immersed in a small bottle containing said substances in liquid form and conveying, by means of capillarity, the essence into the vicinity of a heating element. The present invention relates to this latter type of heating device. In particular it relates to an adjustable-intensity heating device, i.e. in which the intensity of evaporation of the liquid chemical substance may be adjusted within a predetermined range, by varying the quantity of heat transmitted from the heating element to the wick.

In the vast sector of evaporation heating devices, there has been a growing demand for a heating device which offers the possibility of adjustment in accordance with the particular requirements of the user. In fact, however practical and low-cost a standard heating device without adjustment features may be, the possibility of being able to adjust the evaporation intensity undeniably offers a series of considerable advantages.

Firstly, by adjusting this intensity it is possible to modify the quantity of active consituent introduced into the room depending on the particular conditions of the latter: for example, where the room is small or there is insufficient renewal of air, the evaporation intensity must be able to be kept low compared to large or well-ventilated rooms.

Secondly, the quantity of chemical substance introduced into the room may be adjusted depending on the individual sensitivity of each person, making the use of insecticides tolerable even in rooms frequented by persons who find said insecticides particularly bothersome.

Thirdly, with a heating device of this type it is possible to perform an initial intense and hence rapid disinfestation of the insects or scenting of the room and then maintain, over time, the effects of this action with the introduction of a small quantity of active substance.

Therefore, the higher cost of manufacturing a variable-intensity heating device, resulting from its greater constructional complexity, is amply offset by its improved performance.

Various solutions have been proposed hitherto in technology in order to obtain adjustment of the evaporation intensity. Here we shall not consider the attempts aimed at adjusting the heating intensity of the heating element using electrical or electronic methods, since they are completely outside the subject-matter of the present invention.

According to a different approach, the evaporation intensity is varied by adjusting the relative position of the wick and of the heating element, while keeping constant the calorific power produced by the latter. In particular, the surface portion of the wick exposed to the heating element is increased or decreased, thus correspondingly increasing or decreasing the quantity of evaporated chemical substance. This operation, for example, is performed by moving the elements closer together along the longitudinal axis of the wick.

This latter technical solution has assumed essentially two configurations in the prior art, a first one where the heating element is displaced with respect to the wick, and a second one where, on the other hand, the wick is displaced with respect to the heating element.

Normally wick-type heating devices consist of a box-shaped body from which a power supply plug projects and which comprises a heating element and means for receiving and locking in position a small bottle containing the liquid to be evaporated and the associated wick.

The first configuration of the solutions of the known art mentioned above has the drawback, however, that the heating element must be moved with respect to the inside of the box-shaped body and hence also with respect to the power plug, together with the associated connections and electrical contacts. This, in addition to being awkward, makes the entire mechanism delicate and susceptible to accidental breakages.

The known art has resolved this problem by using the second configuration mentioned above, namely by constructing a heating device in which the heating element, together with the associated electrical connections, is integral with the box-shaped body and with the power supply plug, while the bottle with the associated stopper are inserted to a greater or lesser degree inside the box-shaped body by moving the wick closer to the heating element by a varying amount. In ES-U-8800978, for example, a bottle with stopper has been provided, said bottle being able to be inserted manually, more or less fully, into a seat of the box-shaped body by operating, with a finger, an elongated projection formed on one side of the bottle and received in a guiding groove of the seat so as to project outside the heating device.

However, the relative position assumed by the bottle inside the seat, and hence by the wick with respect to the heating element, is ensured only by the friction existing between them. It may therefore easily happen an unwanted extraction of the bottle from the box-shaped body or in any case its displacement from the desired position.

Moreover, in order to be able to check visually the degree of insertion of the bottle inside the box-shaped body, it is necessary to be able to observe the relative position of the elongated projection inside the associated groove of the seat. This involves access to the bottom side part of the heating device, which is not particularly easy when the heating device itself is engaged in a conventional power socket arranged not far above the floor. Therefore, every time the heating device must be adjusted, the user must crouch down into an uncomfortable position or pull out the heating device from the power socket, interrupting operation thereof.

Finally, since sliding of the projection inside the guiding groove is obtained by pressure of a finger, it is neither pleasing nor simple to adjust. Moreover, the force which may be exerted on the projection is very small, such that adjustment becomes awkward or impossible when the friction between the bottle and the associated support bush increases undesirably as a result of contamination or spillage of the liquid substance.

The object of the present invention is to solve the abovementioned drawbacks. In particular, one object is to provide a wick-type heating device in which adjustment of the evaporation intensity is performed with a device which does not allow accidental displacement or extraction of the bottle from the box-shaped body.

A further object is to provide a heating device which, having the abovementioned characteristics, also allows easy and immediate verification of the intensity adjustment in a comfortable position for the user and without interrupting operation of the heating device.

These objects are achieved with a heating device for the evaporation of liquid chemical substances, such as insecticides or perfumed essences, of the type comprising a box-shaped body to which a bottle containing said substance and provided with a wick may be associated, said wick partially facing an electric heating element integral with the box-shaped body, and in which the relative position of the wick and heating element is adjustable, wherein said bottle is fixed to a sleeve sliding inside a bush integral with the bottom part of the box-shaped body, said sleeve comprising an operating arm extending through a window formed on the box-shaped body.

Further characteristic features and advantages of the heating device according to the invention will emerge, however, more clearly from the detailed description which follows of a preferred embodiment thereof, provided by way of example and illustrated in the accompanying drawings, in which:
Fig. 1 is a longitudinally sectioned view of the heating device according to the invention in the minimum supply condition;
Fig. 2 is a sectional view, similar to Fig. 1, with the heating device in the maximum supply condition;
Fig. 3 is a cross-sectional view along the line III-III of Fig. 1 and;
Fig. 4 is a front view of the heating device without the bottle.

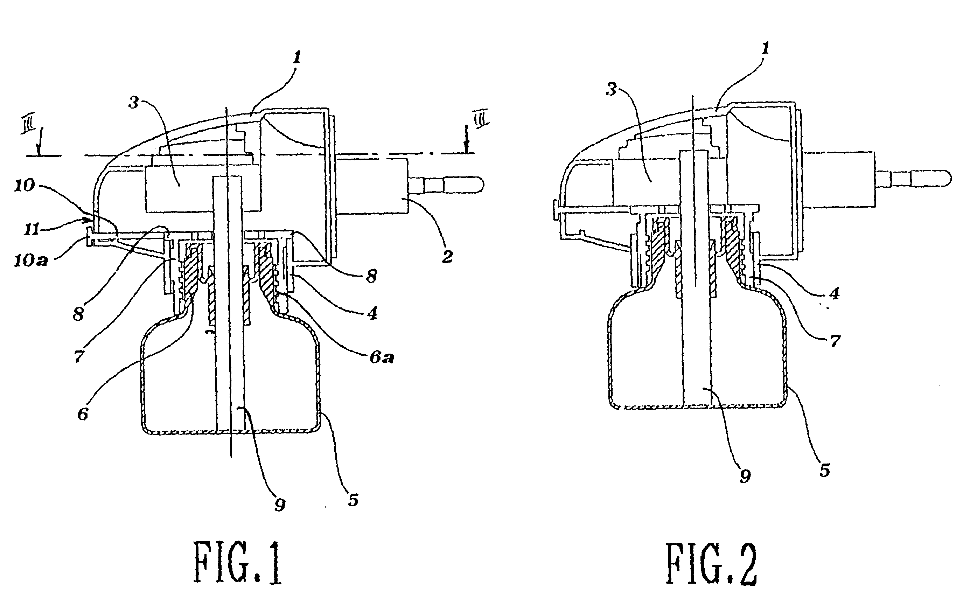

As can be clearly seen in Figs. 1 and 2, the heating device is composed of a box-shaped body 1, from which there projects at the rear (towards the right-hand side in the Figures) an electric power supply plug 2 comprising a heating element 3 supplied electrically by means of electrical connections (not shown) to the power supply plug 2. The box-shaped body 1 has, at the bottom, a seating bush 4 designed for the partial insertion of a small bottle 5 containing the liquid chemical substance to be evaporated.

The bottle 5 has a neck 6 on which a thread 6a is formed. According to the present invention, the neck 6 of the bottle 5 is screwed tightly into a corresponding female thread of a sliding sleeve 7 which is, in turn, housed inside the bush 4.

The external diameter of the sliding sleeve 7 and the internal diameter of the bush 4 are such that these two elements are able to slide one inside the other with a minimum degree of play. Therefore the bottle 5, fixed to the sleeve by means of engagement of the thread 6a in the corresponding female thread, will be able to slide together with the sleeve relative to the box-shaped body 1.

In order to prevent the sliding sleeve 7 from becoming completely detached from the bush 4, according to the present invention, said sleeve has in the region of its top edge a radially projecting profile, for example a circular flange 8, which, coming into contact with the top edge of the bush 4, prevents it from being extracted downwards. Therefore, the sleeve 7 is inserted from above into the bush 4, before the box-shaped body is closed and, subsequently, the bottle 5 is screwed into the latter from below.

The travel of the sleeve in the opposite direction is limited by the presence of the underlying body, which, having a diameter larger than that of the bush, comes into abutment against the walls of the latter when it is situated in its upper position, as shown in Fig. 2.

The neck 6 of the bottle has emerging from it in a known manner a wick 9 which extends into the vicinity of the heating element 3 arranged in a fixed position inside the box-shaped body 1. The wick 9 absorbs the liquid contained in the bottle 5 and transfers it, by means of capillarity, into the vicinity of the heating element so as to allow evaporation thereof. The upper part of the box-shaped body therefore has openings in the form of a grill (shown in Fig. 4) for allowing the evaporated chemical substance to flow out.

As can be seen from a comparison between Figs. 1 and 2, the bottle 2, by means of the sleeve 7, is able to slide between a bottom position (Fig. 1), in which the flange 8 rests on the top edge of the bush 4, and an upper position (Fig. 2), in which the bottle comes up against the bottom edge of the bush 4. The wick 9, accordingly, is displaced together with the bottle between a bottom position, in which only its upper edge is located opposite the heating element 3, and an upper position, in which a sizable section of the wick 9 faces the heating element. It is obvious that the greater the portion of wick exposed to the heating element, the greater will be the quantity of evaporated liquid. Consequently, the evaporation intensity will be minimum when the bottle and the wick are located in the bottom position and maximum when they are located in the upper position, with a whole varying range of evaporation intensities in the intermediate positions.

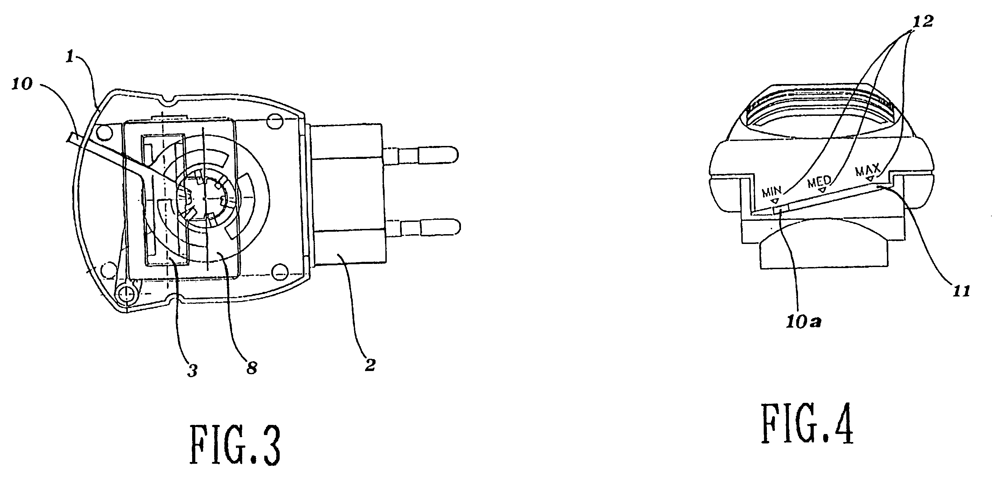

According to the present invention, moreover, the sliding sleeve 7 has an arm 10 (Fig. 3) which extends radially and lies on the same plane defined by the circular flange 8 and which is inserted into a window 11 formed frontally in the thickness of the box-shaped body 1. The window 11 is inclined with respect to the horizontal and therefore guides the end 10a of the arm 10 as if on an inclined surface.

The displacement of the arm 10 therefore causes rotation of the sleeve 7, integral therewith, inside the bush 4 and, at the same time, sliding on the inclined surface of the window 11, draws it upwards or downwards together with the bottle and the wick. The arm 10 can also perform the function of the circular flange 8, i.e. prevent that the bush 4 can be extracted downwards, just cooperating with the walls of the window 11. In this case the arm 10 must have stronger structure, and flange 8 can be omitted.

On the basis of the above description, the window 11 must be designed taking into account two contrasting requirements: on the one hand, its inclination must be sufficiently small to ensure that the friction generated with the arm 10 does not allow the latter to slide along the window itself, being displaced into an unwanted position, not even when a pulling or pushing force is accidentally exerted on the body of the bottle. Moreover the inclination must be sufficiently great to ensure that, between the two ends, there is a vertical height equivalent to the vertical travel which the sleeve 7 performs between its upper position and its bottom position.

As can be seen in Fig. 4, the front external surface of the box-shaped body 1 has designed on it, in the region of the window 11, reference notches 12 for the end 10a of the arm 10, which are able to indicate the relative position assumed by the bottle 5 with respect to the box-shaped body and, indirectly, the relative position of the wick 9 and the heating element 3 and, therefore, ultimately, the evaporation intensity.

The arm 10 therefore has the dual function of achieving the desired adjustment, by means of an easy operation performed by the user, and allowing easy reading of the set evaporation intensity.

The reading of the position of the end 10a with respect to the reference notches 12, since it is performed on the front side of the heating device, may also be clearly visible from above (see Fig. 3) and operation of the arm 10 is also easily performed, without unplugging the heating device itself from the wall socket.

By way of conclusion, the design of the heating device according to the present invention fully achieves the desired objects. On the one hand, it prevents the sleeve, integral with the bottle 5, from being able to become accidentally detached from the bush 4 inside which it slides. On the other hand, it allows comfortable reading of the intensity setting and easy and precise variation in said setting, preventing at the same time any unwanted displacements of the position in which the bottle has been arranged.

It is understood, in any case, that protection of the present invention is not limited to the particular embodiment shown in the drawings, but extends to any other modification thereof, within the scope of the claims indicated below.

## Claims

1. Heating device for the evaporation of liquid chemical substances, such as insecticides or perfumed essences, comprising a box-shaped body to which a small bottle containing said substance and provided with a wick may be associated, said wick partially facing an electric heating element integral with the box-shapad body, and in which the relative position or the wick and heating element is adjustable, **characterized in that** said bottle is fixed to a sleeve sliding inside a fitting bush integral with the bottom part of the box-shaped body, said sleeve comprising an operating arm extending through a window formed on the box-shaped body.

2. Heating device as claimed in Claim 1, wherein said window is formed on the front part of the box-shaped body and is inclined with respect to the horizontal.

3. Heating device as claimed in claim 1), wherein said sleeve comprises, in the region of its upper edge, a radialy projecting stop profile.

4. Heating device as claimed in Claim 3, wherein said radially projecting stop profile is in the form of a circular flange.

5. Heating device as claimed in Claim 4, wherein said arm is coplenar with said circular flange.

6. Heating device as claimed in any one 5 the preceding claims, wherein the bottle is fixed to said sleeve by means of a threaded connection.

7. Heating device as claimed in Claim 6, wherein said threaded connection comprises a female thread on the internal surface of said sleeve and a male thread on the external surface of the neck of the bottle.

## Patentansprüche

1. Heizgerät für die Verdampfung flüssiger chemischer Substanzen, wie Insektiziden oder wohlriechenden Extrakten, das einen kastenförmigen Körper umfaßt, mit dem eine kleine Flasche, die die Substanz enthält und die mit einem Docht ausgerüstet ist, in Verbindung stehen kann, der Docht teilweise einem elektrischen Heizelement, das in den kastenförmigen Körper integriert ist, gegenüber liegt und in dem die relative Stellung des Dochtes und des Heizelementes einstellbar ist, **dadurch gekennzeichnet, daß** die Flasche an einer Manschette befestigt ist, die innerhalb einer Montagebuchse, die in den Bodenbereich des kastenförmigen Körpers integriert ist, gleitet, die Manschette einen Bedienungsarm umfaßt, der sich durch ein Fenster erstreckt, das an dem kastenförmigen Körper gebildet ist.

2. Heizgerät wie in Anspruch 1 beansprucht, bei dem das Fenster auf dem Vorderbereich des kastenförmigen Körpers gebildet ist und in Bezug auf die Horizontale geneigt ist.

3. Heizgerät wie in Anspruch 1 beansprucht, bei dem die Manschette im Bereich ihrer oberen Kante ein radial vorstehendes Stopperprofil umfaßt.

4. Heizgerät wie in Anspruch 3 beansprucht, bei dem das radial vorstehende Stopperprofil in Form eines kreisförmigen Flansches ist.

5. Heizgerät wie in Anspruch 4 beansprucht, bei dem der Arm coplanar mit dem zirkulären Flansch ist.

6. Heizgerät wie in einem der vorangehenden Ansprüche beansprucht, bei dem die Flasche mittels einer Verbindung mit Gewinde an der Manschette befestigt ist.

7. Heizgerät wie in Anspruch 6 beansprucht, bei dem die Verbindung mit Gewinde ein Innengewinde auf der inneren Oberfläche der Manschette und ein Außengewinde auf der äußeren Oberfläche des Flaschenhalses umfaßt.

## Revendications

1. Dispositif chauffant pour l'évaporation de substances chimiques liquides, telles que des insecticides ou des essences parfumées, comprenant un corps en forme de boîtier auquel un petit flacon contenant ladite substance et doté d'une mèche peut être associé, ladite mèche donnant en partie sur un élément chauffant électrique d'un seul tenant avec le corps en forme de boîtier, et dans lequel la position relative de la mèche et de l'élément chauffant est réglable, **caractérisé en ce que** ledit flacon est fixé à un manchon glissant à l'intérieur d'une douille de fixation d'un seul tenant avec la partie inférieure du corps en forme de boîtier, ledit manchon comprenant un bras d'actionnement s'étendant à travers une fenêtre formée sur le corps en forme de boîtier.

2. Dispositif chauffant selon la revendication 1, dans lequel ladite fenêtre est formée sur la partie avant du corps en forme de boîtier et est inclinée par rapport à l'horizontale.

3. Dispositif chauffant selon la revendication 1, dans lequel ledit manchon comprend, dans la région de son bord supérieur, un profil de butée en saillie radiale.

4. Dispositif chauffant selon la revendication 3, dans lequel ledit profil de butée en saillie radiale se présente sous la forme d'un rebord circulaire.

5. Dispositif chauffant selon la revendication 4, dans lequel ledit bras est situé dans le même plan que ledit rebord circulaire.

6. Dispositif chauffant selon l'une quelconque des revendications précédentes, dans lequel le flacon est fixé audit manchon au moyen d'une connexion filetée.

7. Dispositif chauffant selon la revendication 6, dans lequel ladite connexion filetée comprend un filet femelle sur la surface interne dudit manchon et un filet mâle sur la surface externe du col du flacon.
